# EUROPÄISCHE PATENTANMELDUNG

(11) **EP 1 782 847 A2**
(43) Veröffentlichungstag der Anmeldung: **09.05.2007**
(21) Anmeldenummer: 06016761.6
(22) Anmeldetag: 11.08.2006
(51) Int. Cl.: A61L 27/20, A61L 27/52

(54) **Leitschiene für die Regeneration von degenerierten neuronalen Gewebe**

(30) Priorität: 29.08.2005 DE 102005040816
(71) Anmelder: SKH GmbH, 04496 Ortenburg (DE)
(72) Erfinder: Heckmann, Klaus, Dr. Prof., 93186 Pettwendorf (DE); Kunz, Werner, Dr. Prof., 93077 Bad Abbach (DE); Müller, Rainer, Dr., 93053 Regensburg (DE); Prang, Peter, Dr, 93053 Regensburg (DE); Weidner, Norbert, 93053 Regensburg (DE); Eljaouhari, Ahmed, 93053 Regensburg (DE)
(74) Vertreter: Strasse, Joachim

(57) **Zusammenfassung**

Die Erfindung betrifft die ionotrope Herstellung von anisotropen Kapillarengelen zur Verwendung als Leitschiene für nachwachsende Axone bei der Regeneration von degeneriertem neuronalen Gewebe. Zur strukturellen und funktionellen Wiederherstellung geschädigter Axone sowohl im Zentralnervensystem als auch im peripheren Nervensystem wird eine Gerüststruktur benötigt, welche die geordnete Wiederaussprossung durchtrennter Nervenendigungen, deren längsgerichtetes Wachstum über die Strecke der Läsion und damit die Reinnervierung ursprünglicher Zielneurone ermöglicht.

Gerüststrukturen mit den geforderten Eigenschaften lassen sich in an sich Weise aus einer Reihe von Polyelektrolyten mit negativen Festladungen herstellen, wenn man die Sole der Polyelektrolyte durch gerichtete Eindiffusion von Salzlösungen mehrwertiger Kationenionotrop gelieren lässt. Die ionische Quervernetzung verleiht den Gelen eine gewisse mechanische Festigkeit, die sich durch zusätzliche kovalente Quervernetzung noch steigern lässt. Es wird ein Beispiel für die Herstellung von anisotropen Alginatgelen genannt. Die Erfindung besteht in der Dotierung mit Wachstumsstoffen, Zellen usw. und die Verwendung der so modifizierten Gerüststrukturen als Leitschienen.

## Beschreibung

Die Erfindung betrifft eine Leitschiene für die Regeneration von degeneriertem neuronalen Gewebe und ein Verfahren zu seiner Herstellung. (Die Leitschiene in diesem Sinne ist ein Implantat).

Im Hintergrund der vorliegenden Erfindung steht ein Bedarf, Mittel zur Verfügung zustellen, traumatische Querschnittsverletzung zu beheben. Hierfür und für ähnliche Eingriffe werden Leitschienen benötigt, welche eine strukturelle und funktionelle Erholung nach traumatischer Querschnittsverletzung ermöglichen.

Bei einem Rückenmarkstrauma kommt es zum Gewebsuntergang mit irreversiblem Verlust von Glia- und Nervenzellen. Im Gegensatz zu vielen anderen Organen ist das Rückenmark als Teil des Zentralnervensystems nicht in der Lage, dieses zerstörte Gewebe organspezifisch wiederherzustellen. Wesentliche Fortschritte wurden zumindest auf tierexperimenteller Ebene mit zellbasierten Transplantationsstrategien erzielt. Bestimmte Zelltypen ermöglichen, zugrunde gegangenes Rückenmarksgewebe zu ersetzen und als wachstumsförderndes Substrat für wiederaussprossende Nervenendigungen zu dienen. Jedoch regenerieren Nervenendigungen nicht in einer geordneten längsorientierten Art und Weise, was eine gezielte Reinnervierung ursprünglicher Zielneurone verhindert. Eine funktionelle Erholung ist damit ebenfalls ausgeschlossen.

Durchtrennte Axone des peripheren Nervensystems besitzen prinzipiell die Möglichkeit der Regeneration, ausgehend von der proximalen Nervenendigung. In günstigen Fällen erreichen die nachwachsenden Axone ihre Zielneurone, was zu einer Wiederherstellung der ursprünglichen Funktion führen kann. Wird der periphere Nerv im Rahmen einer traumatischen Schädigung komplett durchtrennt, findet in diesem Fall keine erfolgreiche spontane Nervenregeneration statt. Vielmehr wird versucht, ein peripheres Nervenfragment zu implantieren, welches an anderer Stelle ohne erhebliche funktionelle Beeinträchtigung entnommen werden kann (Nerv-Autotransplantat). Die Verfügbarkeit solcher Nerv-Autottransplantate ist dabei äußerst begrenzt.

Deshalb wird zur strukturellen und funktionellen Wiederherstellung geschädigter Axone sowohl im Zentralnervensystem als auch im peripheren Nervensystem eine Gerüststruktur, nämlich eine Leitschiene benötigt, welche eine geordnete Wiederaussprossung durchtrennter Nervenendigungen ermöglicht. Hierzu ist ein längsgerichtetes Wachstum der Axone über die Strecke der Läsion und damit die Reinnervierung ursprünglicher Zielneurone erforderlich. Diese Gerüststruktur sollte darüber hinaus die Inkorporierung geeigneter Zelltypen und Wachstumsmoleküle ermöglichen, welche die Regenerationsfähigkeit verletzter Nerven verbessern. Weiterhin soll die Gerüststruktur die für das "Tissue Engineering" geforderte Biokompatibilität und temporäre Stabilität (Biodegradierbarkeit) aufweisen. Als besonders geeignete Gerüststrukturen bieten sich anisotrope Kapillarenbündel aus geeignetem Material an.

Die WO - Schrift 03/026489 A2 beschreibt zum Beispiel die Verwendung von Kompositionen, die neben zellulären Strukturen und Wachstumsfaktoren ein Biomaterial in Form eines quervernetzten Gels oder eines mikroporösen Schaums, bestehend aus Alginat, Hyaluronsäure, Chitosan, Agarose, Carboxymethylcellulose oder deren Mischungen, enthält. Dieses Trägermaterial weist aber aufgrund der Herstellungsbedingungen keine anisotrope Kapillarenstruktur auf und ist somit keine längsgerichtete Leitschiene.

Die WO - Schrift 98/16266 beschreibt die Verwendung von Hydrogelen aus quervernetzbaren N-substituierten Methacrylamiden oder Acrylamiden in Verbindung mit Kohlenhydratderivaten, Adhäsionspeptiden und Antikörpern. Diese Hydrogele weisen ebenfalls keine anisotrope Kapillarenstruktur auf.

Die WO - Schrift 96/02286 beschreibt die Herstellung und Verwendung einer künstlichen extrazellulären Matrix in Form eines Hydrogels, welches aus einem Kohlenhydrat, beispielsweise Agarose, besteht und mit einem Adhäsionspeptid modifiziert ist und welches in das Lumen eines "nerve guidance channels" eingebracht werden kann. Diese Hydrogele weisen allerdings auch keine anisotrope Kapillarenstruktur auf.

Im zentralen Nervensystem, speziell im Rückenmark wurden als Implantate die folgenden Materialien verwendet: Kollagen, Milliporefilter, Kohlenstofffasern, Fibrin, Laminin und Fibronektin. Auch diese Materialien weisen keine anisotrope Kapillarenstruktur auf, wie sie für eine gerichtete Regeneration der beschädigten Axone notwendig ist.

Gemäß der WO - Schrift 2005046457 werden Fasern aus festem Material als Negative für die Ausbildung von Poren verwendet. Die herstellbaren Porendurchmesser liegen zwischen 50 und 500 µm, sind folglich zumindest im oberen Bereich für die zielgerichtete Regeneration von Axonen zu groß. Negativ wirkt sich auch aus, dass die Faseroberflächen mit "Mikrodomänen" ausgestattet sind, die zur Aufnahme von wachstumsfördernden Substraten dienen sollen. Hierdurch wird in jedem Fall die Oberfläche der Negativ-Fasern aufgeraut, was beim Herausziehen der Fasern aus dem Gel am Ende des Fertigungsprozesses zwangsläufig zu noch größeren Verletzungen der Kapillarenwände führen muss, als bei der nachstehen abgehandelten WO 99/11181. Die Anfälligkeit für Verletzungen und Zerstörungen auf diesem Weg nimmt natürlich mit sinkenden Porendurchmessern zu. Sie ist also dort besonders groß, wo sich die Kapillaren für die zielgerichtete Regenerierung am besten eignen sollten.

Die vorliegende Erfindung geht von einem Stand der Technik aus, wie er in der WO - 99/11181 beschrieben ist. Im Wesentlichen wird hier der Gedanke vorgestellt, Bündel von parallel geordneten Kapillaren als Leitstrukturen für das gerichtete regenerative Wachstum von Axonen auf handwerkliche Weise herzustellen. Die WO 99/11181 beschreibt einen Kanal oder Leiter zur Nervenregenerierung, der aus Polymer-Material durch mechanische Handhabung extrudiert oder gegossen wird (Seite 6 ab Zeile 25), was allerdings die Anpassung an inviduelle Gegebenheiten unmöglich macht.

Es wird in der WO - Schrift 99/11181 als Alternative vorgeschlagen, ein geordnetes Bündel von Drähten als Negativ in eine Gussform einzubringen und dann zwischen und um diese Drähte herum ein Hydrogel zu gießen und erstarren zu lassen. Die auf diese Weise herstellbaren Porendurchmesser sollen zwischen 2 und 500 µm liegen. Diese Porendurchmesser sind allerdings nur im aller untersten Bereich so klein, dass sie den aussprossenden Axonen eine hinreichend zielsichere Leitstruktur bieten können. Darum wirkt sich genau an dieser Stelle das Herstellungsverfahren als besonders unhandlich und kostspielig aus: Jeder der als Negativ vorgegebenen Metalldrähte muss zunächst einzeln positioniert und am Ende des Verfahrens einzeln wieder aus seiner Form und dem Gel herausgezogen werden. Diese Schrift beschränkt sich auf die Aussage, dass die dort verwendeten Polymere sich unter den gewählten Versuchsbedingungen als biokompatibel erweisen. Gegenüber diesem Stand der Technik ist es die verfeinerte Aufgabe der Erfindung, ein Führungssysten verfügbar zu machen, welches keinen Rückgriff auf ein mechanisch vorgefertigtes Hilfsmittel benötigt, sondern einem natürlichen Regenerationwachstum eine organisch - chemisch gewachsene, gerichtete Bahn vorgibt.

Die Lösung dieser Aufgabe ergibt sich aus dem Hauptanspruch.

Die Tatsache, dass unter bestimmten experimentellen Bedingungen so genannte ionotrope Gele mit einer geordneten, anisotropen Kapillarenstruktur gebildet werden, wenn ein Sol aus Natriumalginat mit einer Lösung eines mehrvalenten Kations in Kontakt tritt, ist prinzipiell aus DE 1 011 853 A1 für technische Anwendungen bekannt (Heinrich Thiele). Dies geschieht aber nur, wenn beide Flüssigkeiten ohne die Erzeugung von Konvektion über eine horizontal verlaufende Phasengrenze miteinander in Berührung stehen. Beim ersten Kontakt der beiden Flüssigkeiten bildet sich in der Grenzzone zwischen den beiden Flüssigkeiten eine dünne Membran (Primärmembran) aus. Diese besteht aus durch die hinzu gegebenen Kationen quervernetztem Alginat (z.B. Cu-Alginat oder Ca-Alginat), was von Thiele als ionotrope Gelbildung bezeichnet wurde. Durch diese dünne Membran können nun Kationen und Anionen des Salzes hindurch diffundieren, nicht aber die Alginatketten. Während der gerichteten Diffusion der Metallkationen in einer Richtung bildet sich durch stetige Quervernetzung ein Gel aus, welches nur unter diesen bestimmten Bedingungen eine anisotrope Struktur mit parallel verlaufenden Kapillaren aufweist. Gemäß DE 1 011 853 A1 bezogen sich die Lösungsansätze auf rein technische Anwendungen, wie sie später beispielsweise in der US Patentschrift 3,350,232 für die Ausgestaltung von dünnen Zwischenplatten in Akkumulatoren aufgegriffen wurden.

Alle anderen Methoden, Alginathydrogele herzustellen, führen nicht zur Ausbildung von gerichteten Kapillaren. Man erhält stattdessen nur isotrope Strukturen. Diese, vorwiegend Ca-Alginat, können allerdings auf eine lange Tradition in der Biomedizin zurückblicken, wo sie als Wundauflage, als injizierbare Zellträger (Mikrokapseln) und auch als Träger- bzw. Depotstruktur für Medikamente ("drug delivery") eingesetzt werden.

Gerüststrukturen mit den geforderten Eigenschaften, nämlich eine gewünschte Wachstumsgrößen- und -richtung vorzugeben und seitliche Abweichungen zu vermeiden, lassen sich dagegen erfindungsgemäß aus einer Reihe von Polyelektrolyten mit negativen Festladungen herstellen, wenn man die Sole der Polyelektrolyte durch gerichtete Eindiffusion von Salzlösungen mehrwertiger Kationen ionotrop gelieren lässt. Die ionische Quervernetzung verleiht den Gelen eine gewisse mechanische Festigkeit, die sich durch zusätzliche kovalente Quervernetzung noch steigern lässt. Da sich die Alginatgele wie lonenaustauscher verhalten, können die für die Quervernetzung verantwortlichen mehrwertigen Kationen quantitativ ausgetauscht werden. Dies führt bei einem längeren Kontakt mit biologischen Medien zu einer Auflösung des strukturierten Gels, da die quervernetzenden Ionen durch einwertige, nicht quervernetzende Kationen ersetzt werden. Eine signifikante Verbesserung der biologischen Stabilität der strukturierten Hydrogele konnte durch eine chemische Quervernetzung mit Z.B. Hexamethylendüsocyanat (HDI) erreicht werden. In Vorversuchen konnten derart stabilisierte Strukturen drei Wochen lang in den spinalen Defekt einer Ratte implantiert werden, ohne dass Hinweise auf eine signifikante Degradation erkennbar waren. Zudem waren keine toxischen Reaktionen nachweisbar. Weitere Methoden zur chemischen Quervernetzung der anisotrop strukturierten Kapillargele sind die Verwendung von Dialdehyden, wie z.B. Glutaraldehyd, oder von Carbodiimiden in Verbindung mit Diaminen, oder von disulfidhaltigen Verbindungen, wie Dithiodicarbonsäuren, in Verbindung mit Carbodiimiden.

Enthält das Polyelektrolytsol noch suspendierte Feststoffpartikel, so finden sich diese nach der Gelbildung in den Kapillarenwänden wieder, was nochmals die mechanische Stabilität der Gele erhöht. Festkörperhaltige Kapillarengele lassen sich rissfrei zu Grünkörpern trocknen und danach mechanisch formgebend verarbeiten. Auf diese Weise kann man maßhaltige Implantate von gegebener Größe und Form herstellen. Nach der Formgebung werden die Feststoffpartikel aus dem Gel vorsichtig herausgelöst. Beispiele für oben genannte Polyelektrolyte sind Alginate, Pektinate, Carboxymethylcellulosen, Hyaluronsäure sowie weitere anionische Glycosaminoglycane und anionisch derivatisierte Kohlenhydrate. Beispiele für die zur Vernetzung verwendeten mehrwertigen Kationen sind die zweiwertigen Ionen des Kupfers, des Calciums, des Bleis; Beispiele für geeignete suspendierte Feststoffe sind eine Reihe von säurelöslichen Metalloxiden oder - hydroxiden, wie z.B. Calciumhydroxid, oder Metallphosphate, wie z.B. Tricalciumphosphat.

Die Kapillarenwände der nach der neuen Verwendung gemäß einem Verfahren aus anisotrop strukturierten Hydrogele hergestellt, die mit Proteinen der extrazellulären Matrix beschichtet werden, die einen chemotaktischen Reiz auf zelluläre Strukturen ausüben, die sich in den Lumina der Kapillaren befinden. Dies führt zu einer verbesserten Zelladhäsion auf der Gerüststruktur aber auch zu einer verstärkten Proliferation, Differenzierung oder Migration der Zellen. Als Beispiele für Proteine der extrazellulären Matrix können Kollagen, Fibrinogen, Fibronektin und Laminin angeführt werden.

Die Kapillarenwände werden nach einem an sich bekannten Verfahren für die vorliegende Anwendung modifiziert hergestellt, wobei die anisotrop strukturierten Hydrogele mit neurotrophen Faktoren und/oder Wachstumsfaktoren werden Als Beispiele hierfür können nerve growth factor (NGF), basic fibroblast growth factor (bFGF), brain-derived neuro- notrophic factor (BDNF), ciliary neuronotrophic factor (CNTF), glial cell line-derived neurotrophic factor (GDNF), neurothrophins (NT-3 and NT-4/5), oder glial growth factor (GGF) angeführt werden. Die Freisetzung dieser Faktoren kann durch Diffusion aus den Kapillarenwänden nach direkter Inkubation des Hydrogels in Lösungen des Faktors oder nach Inkorporation anderer Freisetzungssysteme, wie z.B. Mikrokapseln, erfolgen.

Nach einem Ausführungsbeispiel für die Herstellung von anisotropen Alginatgelen wird Natriumalginat (Manugel von Kelco, Pronova von NovaMatrix oder vergleichbare Produkte) in einer Konzentration von 1 bis 2 % (10 bis 20 mg/ml) in reinem Wasser gelöst, wobei diese Polyelektrolytlösung als Sol bezeichnet wird. Dieses Sol wird luftblasenfrei in eine geeignete Glas- oder Aluminiumform überführt. Die Oberfläche des Sols wird mit Hilfe von Sprühflaschen oder ähnlichem mit einer wässrigen Lösung eines geeigneten Salzes gleichmäßig und ohne die Erzeugung von Konvektion überschichtet. Die wässrige Salzlösung beinhaltet eine Sorte eines mehrwertigen Kations (z.B. Ca₂+, Cu₂+, Al₃+, La₃+, usw.) und eine Sorte eines geeigneten Anions (z.B. Cl-, NO₃-, usw.) in einer Konzentration an mehrwertigem Kation zwischen 0,1 und 5 mol/l.

Nach der Überschichtung des Sols mit einer ausreichenden Menge an wässriger Salzlösung wird der gesamte Ansatz für 1 bis 5 Tage in Ruhe stehen gelassen. Durch die Variation der kolloidchemischen Parameter kann der Durchmesser der röhrenförmigen Poren im Alginatgel zwischen 10 und 200 µm eingestellt werden. Die gebildeten Gelkörper werden mit Hilfe einer speziell entwickelten Säge durch parallele Schnitte in Gelscheiben definierter Dicke geschnitten. Die Schnitthöhe innerhalb eines Gelkörpers erlaubt ebenfalls eine Variation der Kapillarendurchmesser. Die Gelscheiben werden durch Tauchen in Bädern des organischen aber mit Wasser mischbaren Lösungsmittels Aceton dehydratisiert. Nach vollständiger Entwässerung werden die Gelscheiben zur chemischen Stabilisierung mit in Aceton gelöstem Hexamethylendüsocyanat (HDI) quervernetzt. Überschüssiges HDI wird in weiteren Tauchbädern aus Aceton entfernt.

Zuletzt wird in Tauchbädern aus Wasser das stabilisierte Gel wieder rehydratisiert. Alternativ kann - ohne vorangegangene Entwässerung - auch mit Carbodiimiden/Diaminen querverntzt werden. Der Neurologe/Neurochirurg schneidet mit Hilfe eines Skalpells aus der hergestellten Gelscheibe ein für den Anwendungsort geeignetes Gelstück heraus.

Die nach den obigen Verfahren hergestellten und modifizierten anisotrop strukturierten Hydrogele können mit Zellen in vitro besiedelt werden. Als Beispiele für geeignete Zellen können adulte neurale Stammzellen oder differenzierte Zellen, wie z.B. Schwann Zellen angeführt werden.

Die nach obigen Verfahren hergestellten und modifizierten anisotrop - strukturierten Hydrogele werden mit oder ohne vorherige Besiedelung mit Zellen (adulte neurale Vorläuferzellen, Schwann Zellen) zur Regeneration von Axonen im zentralen und peripheren Nervensystem verwendet.

Die axonale Regeneration nach spinalem Trauma ist prinzipiell möglich. Geführt durch eine geeignete Leitstruktur verläuft die Regeneration gerichtet und führt zur funktionellen Wiederherstellung nach axonaler Schädigung.

Versuche zeigen, dass spezifisch markierbare komplett durchtrennte Nervenbahnen als Bündel gerichtet aus dem entorhinalen Kortex in Streifen anisotroper Kapillargele sprossen und diese durchwachsen und gewinnen am distalen Kapillarende Wiederanschluss an den hippocampalen Anteil der Schnittkultur. Das gute Ergebnis lässt sich nach immunhistochemischer Doppelfärbung mit dem Regenerationsmarker GAP-43 mittels eines konfokalen Laser-Mikroskops sichtbar machen.

Ferner kann man mit dem Regenerationsmarker GAP-43 immunhistochemisch dargestellte wieder aussprossende Axone zeigen, welche der Längsorientierung der Kapillargele folgen. In einer entsprechenden Darstellung erkennt man das implantierte Alginatgel, welches im verletzten Rückenmark einer Ratte durch hochauflösende Kernspintomographie in situ dargestellt wurde. Es zeigt sich eine ausreichende Stabilität des Hydrogels ohne wesentliche Ödem- oder Zystenbildung um das Implantat herum. Immunohistochemische Färbungen zur Darstellung von Makrophagen oder reaktiver Astroglia ergaben keine Hinweise für nennenswerte entzündliche Reaktionen.

Die gerichtete Regeneration durch die Struktur der anisotropen Kapillarengele konnte sowohl an Schnittkulturen in vitro als auch nach spinalem Trauma am Tiermodell der Ratte gezeigt werden

## Patentansprüche

1. Verfahren zur Herstellung einer Leitschiene für die Regeneration von degeneriertem neuronalen Gewebe, wobei ionotrope Gele mit einer geordneten, anisotropen Kapillarenstruktur gebildet werden, in dem ein Sol aus Natriumalginat mit einer Lösung eines mehrvalenten Kations in Kontakt tritt und beide Flüssigkeiten ohne die Erzeugung von Konvektion über eine horizontal verlaufende Phasengrenze miteinander in Berührung stehen sowie beim ersten Kontakt der beiden Flüssigkeiten sich in der Grenzzone zwischen den beiden Flüssigkeiten eine dünne Membran (Primärmembran) bildet, **dadurch gekennzeichnet, dass** sich während der gerichteten Diffusion von Metallkationen in eine Richtung durch stetige Quervernetzung ein Gel ausbildet, das eine anisotrope Struktur mit parallel verlaufenden Kapillaren aufweist, die aus einer Reihe von Polyelektrolyten mit negativen Festladungen hergestellt werden, indem die Sole der Polyelektrolyte durch gerichtete Eindiffusion von Salzlösungen mehrwertiger Kationen lonotrop geliert.

2. Verfahren nach Anspruch 1, **dadurch gekennzeichnet, dass** die ionische Quervernetzung den Gelen eine gewisse mechanische Festigkeit verleiht, die durch zusätzliche kovalente Quervernetzung gesteigert wird.

3. Verfahren nach Ansprüchen 1 und 2, **dadurch gekennzeichnet, dass** die Alginatgele als Ionenaustauscher wirken, um die für die Quervernetzung verantwortlichen mehrwertigen Kationen quantitativ auszutauschen.

4. Verfahren nach Ansprüchen 1 bis 3, **dadurch gekennzeichnet, dass** eine chemische Quervernetzung mit Hexamethylendiisocyanat (HDI) bei einem längeren Kontakt mit biologischen Medien die biologische Stabilität der strukturierten Hydrogele verbessert.

5. Verfahren nach Ansprüchen 1 bis 4, **dadurch gekennzeichnet, dass** zur chemischen Quervernetzung der anisotrop - strukturierten Kapillargele Dialdehyde, wie z.B. Glutaraldehyd, oder von Carbodiimide in Verbindung mit Diaminen, oder disulfidhaltige Verbindungen, wie Dithiodicarbonsäuren, in Verbindung mit Carbodiimiden verwendet werden.

6. Verfahren nach Ansprüchen 1 bis 5, **dadurch gekennzeichnet, dass** das Polyelektrolytsol suspendierte Feststoffpartikel enthält.

7. Verfahren nach einem oder mehren der Ansprüchen 1 bis 6, **dadurch gekennzeichnet, dass** als Polyelektrolyte Alginate, Pektinate, Carboxymethylcellulosen, Hyaluronsäure sowie weitere anionische Glycosaminoglycane und anionisch derivatisierte Kohlenhydrate eingesetzt werden.

8. Verfahren nach einem oder mehren der Ansprüchen 1 bis 6, **dadurch gekennzeichnet, dass** zur Vernetzung der mehrwertigen Kationen die zweiwertigen Ionen des Kupfers, des Calciums, des Bleis verwendet werden.

9. Verfahren nach einem oder mehren der Ansprüchen 1 bis 6, **dadurch gekennzeichnet, dass** als geeignete suspendierte Feststoffe eine Reihe von säurelöslichen Metalloxiden, wie z.B. hgo eingesetzt werden.

10. Verfahren nach einem oder mehren der Ansprüchen 1 bis 9, **dadurch gekennzeichnet, dass** Suspensionen feinverteilter säurelöslicher Feststoffe aus Metallhydroxiden, benutzt werden, wie z.B. Fe(OH)₃, Al(OH)₃, Mg(OH)₂, Cu(OH)₂.

11. Verfahren nach einem oder mehren der Ansprüchen 1 bis 9, **dadurch gekennzeichnet, dass** Suspensionen feinverteilter säurelöslicher Feststoffe aus Metallphosphaten, benutzt werden, wie z.B., Ca₃(P0₄)₃ oder Hydroxylapatit.

12. Verfahren nach einem oder mehren der Ansprüchen 1 bis 11, **dadurch gekennzeichnet, dass** die Kapillarenwände durch chemische Quervernetzung, z.B. durch Diisocyanate, durch Dialdehyde, durch Carbodiimide in Verbindung mit Diaminen, durch disulfidhaltige Verbindungen, wie Dithiodicarbonsäuren in Verbindung mit Carbodiimiden stabilisiert werden, wobei die Produkte der chemischen Quervernetzung hydrolytisch oder reduktiv, speziell enzymatisch katalysiert, aber verlangsamt zum nicht quervernetztem Zustand wieder spaltbar sind.

13. Verfahren nach einem oder mehren der Ansprüchen 1 bis 12, **dadurch gekennzeichnet, dass** die Kapillarenwände der hergestellten anisotrop strukturierten Hydrogele mit Proteinen der extrazellulären Matrix beschichtet werden, die einen chemotaktischen Reiz auf zelluläre Strukturen ausüben, die sich in den Lumina der Kapillaren befinden, was zu einer verbesserten Zelladhäsion auf der Gerüststruktur aber auch zu einer verstärkten Proliferation, Differenzierung oder Migration der Zellen führen.

14. Verfahren nach Anspruch 13, **dadurch gekennzeichnet, dass** als Proteine der extrazellulären Matrix werden Kollagen, Fibrinogen, Fibronektin und Laminin benutzt werden.

15. Verfahren nach einem oder mehren der Ansprüchen 1 bis 14, **dadurch gekennzeichnet, dass** die Kapillarenwände der anisotrop strukturierten Hydrogele mit neurotrophen Faktoren und/oder Wachstumsfaktoren modifiziert werden.

16. Verfahren nach Anspruch 15, **dadurch gekennzeichnet, dass** nerve growth factor (NGF), basic fibroblast growth factor (bFGF), brain-derived neurotrophic factor (BDNF), ciliary neuronotrophic factor (CNTF), glial cell line-derived neurotrophic factor (GDNF), neurothrophins (NT-3 and NT-4/5), oder glial growth factor (GGF) benutzt werden, wobei die Freisetzung dieser Faktoren durch Diffusion aus den Kapillarenwänden nach direkter Inkubation des Hydrogels in Lösungen des Faktors oder nach Inkorporation anderer Freisetzungssysteme, wie z.B. Mikrokapseln, erfolgen.

17. Verfahren nach einem oder mehren der Ansprüchen 1 bis 16, **dadurch gekennzeichnet, dass** für die Herstellung von anisotropen Alginatgelen Natriumalginat (Manugel von Kelco, Pronova von NovaMatrix oder vergleichbare Produkte) in einer Konzentration von 1 bis 2 % (10 bis 20 mg/ml) in reinem Wasser gelöst wird, wobei diese Polyelektrolytlösung als Sol entsteht und dieses Sol luftblasenfrei in eine geeignete Glas- oder Aluminiumform überführt wird, worauf die Oberfläche des Sols mit Hilfe von Sprühflaschen oder ähnlichem mit einer wässrigen Lösung eines geeigneten Salzes gleichmäßig und ohne die Erzeugung von Konvektion überschichtet wird..

18. Verfahren nach Anspruch 17, **dadurch gekennzeichnet, dass** die wässrige Salzlösung eine Sorte eines mehrwertigen Kations (z.B. Ca₂+, Cu₂+, Al₃+, La₃+, usw.) und eine Sorte eines geeigneten Anions (z.B. Cl-, NO₃-, usw.) in einer Konzentration an mehrwertigem Kation zwischen 0,1 und 5 mol/l. beinhaltet.

19. Herstellung von Kapillarengelen gemäß einem oder mehreren der voraufgehenden Ansprüchen, **dadurch gekennzeichnet, dass** deren Kapillarenwände durch physikalische Quervernetzung, z.B. durch Verwendung eines kationischen Polyelektrolyten, wie z.B. Chitosan oder Poly-L-Lysin, usw. stabilisiert werden.

20. Herstellung modifizierter Kapillarengele gemäß einem oder mehrerer der voraufgehenden Ansprüche, **dadurch gekennzeichnet, dass** deren Kapillarenwände mit regenerationsfördernden molekularen Stoffen, wie z.B. Ernährungsmolekülen oder Wachstumsfaktoren in verkapselter oder unverkapselter Form versehen sind.

21. Herstellung modifizierter Kapillarengele gemäß einem oder mehreren der voraufgehenden Ansprüchen, **dadurch gekennzeichnet, dass** deren Kapillarenwände mit regenerationsfördernden zellulären Strukturen, wie z.B. pluripotente Stammzellen, bevorzugt neuronalen Stammzellen oder differenzierten neuronalen Zellen, wie z.B. Gliazellen oder Schwann-Zellen in vitro besiedelt und/oder vorkultiviert sind.
